# EUROPEAN PATENT SPECIFICATION

(11) **EP 0 708 920 B1**
(45) Date of publication and mention of the grant of the patent: **17.06.1998**
(21) Application number: 94924763.9
(22) Date of filing: 13.07.1994
(51) Int. Cl.: G01N 33/28

(54) **A METHOD AND APPARATUS FOR TESTING THE THERMAL STABILITY OF AN (AVIATION) FUEL**
VERFAHREN UND GERÄT ZUR PRÜFUNG DER THERMISCHEN BESTÄNDIGKEIT EINES (FLUGZEUG-) KRAFTSTOFFES
PROCEDE ET APPAREIL DE TEST DE LA STABILITE THERMIQUE D'UN CARBURANT D'AVIATION

(30) Priority: 14.07.1993 EP 93305534
(43) Date of publication of application: 01.05.1996
(73) Proprietor: SHELL INTERNATIONALE RESEARCH MAATSCHAPPIJ B.V., 2596 HR Den Haag (NL)
(72) Inventor: CLARK, Richard, Hugh, Cheshire CH1 3SH (GB); KENDALL, David, Roy, Cheshire CH1 3SH (GB); HOULBROOK, Geoffrey, Cheshire CH1 3SH (GB); HARDWICK, Stewart, Leonard, Cheshire CH1 3SH (GB)
(86) International application number: EP9402324
(87) International publication number: WO9502821

(56) References cited:
- GB-A- 1 252 299
- US-A- 3 670 561
- US-A- 5 101 658
- US-A- 5 162 235
- US-A- 5 299 449

## Description

The present invention relates to a method and apparatus for testing the thermal stability of a fuel, e.g. aviation gas-turbine or jet fuel.

In particular, the invention relates to small scale turbulent flow testing of (jet) fuel thermal stability.

Modern aircraft subject aviation turbine fuel to a range of thermal stresses. This results from the use of fuel as a sink for waste heat and the fact that certain components of the fuel system are located in regions of high ambient temperature. The heat thus acquired by the fuel stimulates oxidation reactions that lead to the formation of insoluble material. This is clearly undesirable and potentially harmful if, for example, lacquers form on heat exchanger and control surfaces, thereby reducing their efficiency.

With respect to heat/thermal stressing of fuel the following points are of interest:
a) The heat stress in aircraft and test rigs is under pressure (greater than 20 atmospheres) to ensure that the fuel does not vaporise but always remains in the liquid phase.
b) Temperatures in aircraft and test rigs are high enough for fuel oxidation reactions to predominate, but not high enough for fuel pyrolysis reactions to predominate.
(c) Heat stressing fuel in a real aircraft will take the fuel to 130-150 °C in the hottest heat exchanger (the oil-cooler) and to 180-200 °C in the feed-arm just prior to the atomiser in the combustion zone.

In addition, the fuel insolubles can obstruct or even block parts of the fuel system, for example, the atomizer head in the combustion zone.

The continuing trend toward higher engine operating temperatures means that there is an increasing likelihood of problems being encountered as a result of thermally induced deposit formation.

A small-scale test configuration has already been proposed, wherein smooth small-bore tubes are applied, which are electrically heated, and under turbulent flow conditions similar to that which is produced in the engine heat exchangers.

However, these known test rigs require large fuel flows and quantities (e.g. 2000-80000 litres of fuel per test).

Further, in the known test rigs the outer wall temperature or thermal resistance of the tube is established to gauge the quantity of fuel lacquer deposited. In these known test rigs a single or two thermocouple measurements are used, thus obtaining a single measure of temperature or some average figure.

However, fuels will not lay down lacquer layers of even thickness along the length of the test tube, but will produce a distribution of lacquer thickness.

Moreover, this pattern of lacquer distribution is fuel dependent and unpredictable. The above systems fail to recognize the important point of lacquer distribution and as a result they could fail to distinguish two fuels of different stability.

Thus, in order to avoid operational problems, it is essential to have a realistic test method and apparatus.

It is an object of the present invention to provide a method and small-scale apparatus for testing the thermal stability of a fuel under turbulent flow conditions, wherein the fuel flow is reduced, e.g. to about 5 litres per test only, which is useful as often in reality only small samples are available.

It is another object of the invention to provide a method and apparatus for assessing a liquids heat stability in a turbulent flow regime, which also recognises and allows the full lacquer pattern to be determined.

The invention therefore provides a method for testing the thermal stability of a fuel comprising the steps of
a) supplying under pressure a fuel sample to be tested to a small-bore capillary tube of a test section and allowing the fuel in the test section to be heat stressed;
b) ensuring that the fuel sample in the test section is under turbulent flow regime;
c) monitoring the thermal degradation of the test fuel within the section by measuring by means of contactless thermometry the outer wall temperatures along the test section and subsequent increases in these temperatures, promoted by the insulation effect of fuel degradation products; and
d) deriving from the temperature data, thus obtained, information on the thermal stability of the fuel.

The invention further provides an apparatus for testing the thermal stability of a fuel comprising means for supplying under pressure a fuel sample to be tested to a small-bore capillary tube of a test section and means for allowing the fuel in the test section to be heat stressed; means for ensuring that the fuel sample in the test section is under turbulent flow regime; means for measuring by means of contactless thermometry the outer wall temperatures along the test section and subsequent increases in these temperatures, promoted by the insulation effect of fuel degradation products; and means for deriving from the temperature data, thus obtained, information on the thermal stability of the fuel.

Reference is made to USA patent specification No. 5 162 235 disclosing a method for assessing distillate fuel wherein a sample of the distillate fuel is heated in the presence of oxygen to a predetermined temperature for a predetermined period in order that degradation products are formed. Thereafter the degradation products are removed and weighed to assess the long term storage stability of the distillate fuel. Reference is further made to USA patent specification No. 5 101 658 disclosing determining the thermal stability of fluids such as fuel, wherein the fluid is passed through a heated conduit at a predetermined rate, and wherein the amount of degradation products collected on a filter is determined to assess the thermal oxidation tendencies of the tested fluid. And reference is made to UK patent specification No. 1 252 299 disclosing testing liquid hydrocarbons, such as jet fuel for their depositforming tendencies, wherein a sample of the liquid is passed at a predetermined rate and for a predetermined period through a heated tubular vessel in which metal strips are placed. The amount of material deposited on the metal strips is then used to assess the depositforming tendency of the tested liquid hydrocarbon. These publications are not relevant to the present invention.

Advantageously, the contactless thermometry is controlled scanning I.R. (infra-red) thermometry.

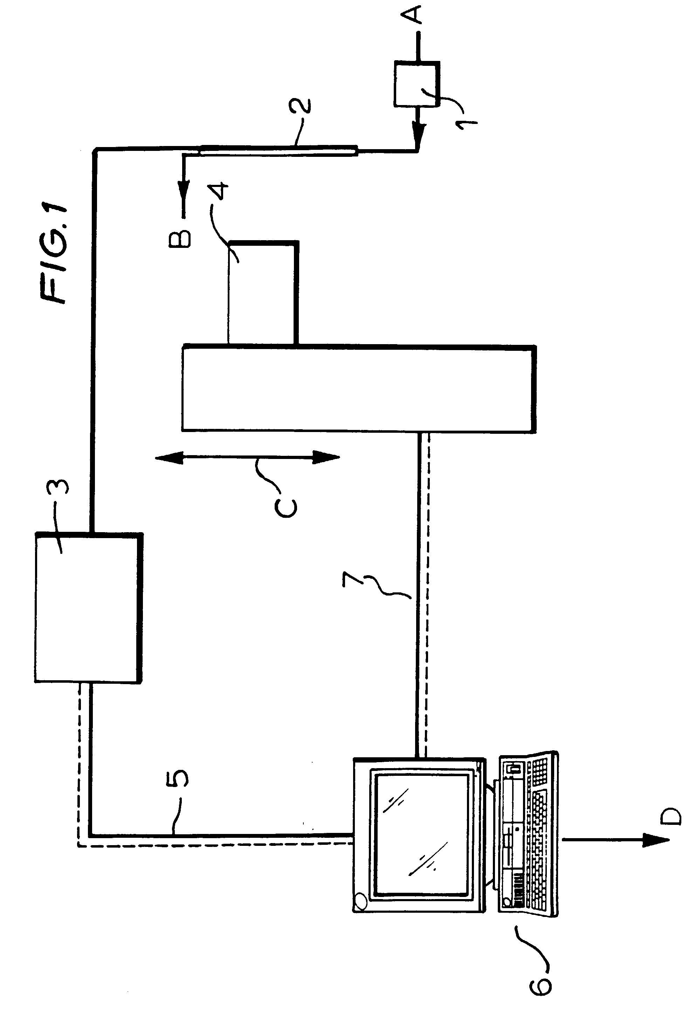

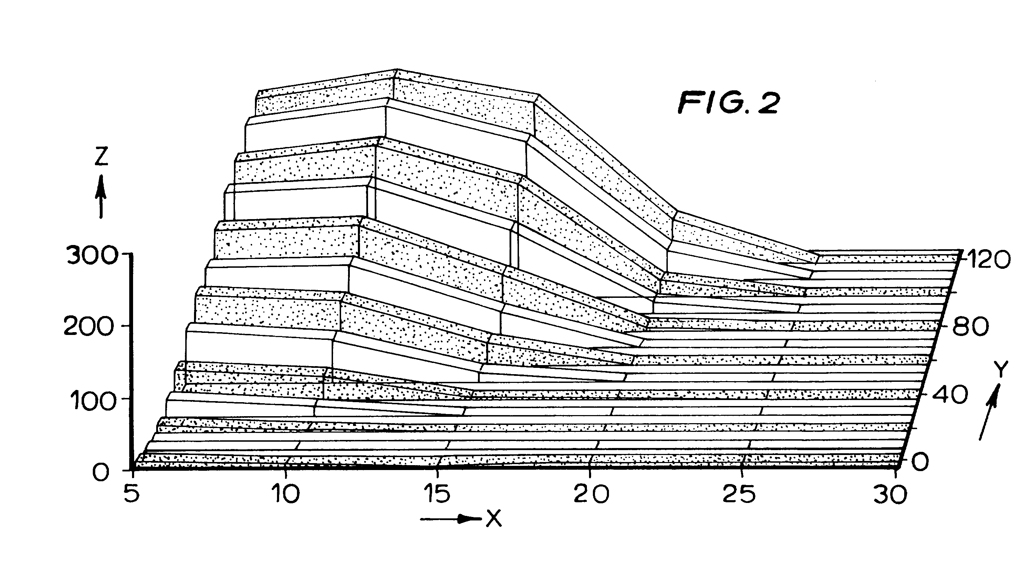

The invention will now be described in more detail by way of example by reference to the accompanying drawings, in which fig. 1 represents schematically an embodiment according to the present invention; and fig. 2 represents a three dimensional temperature profile of the capillary test section, obtained according to the invention.

Referring now to fig. 1, a capillary test section 1 is shown.

Fuel A is supplied in any way suitable for the purpose under pressure by a pump 1 to the capillary test section 2. The test section 2 is e.g. clamped between two electrical bus bars (not shown for reasons of clarity) which supply current and allow the fuel in the test section to be heat stressed; the system is controlled to a constant fuel outlet temperature by any suitable fuel temperature control 3. B represents the outlet fuel stream. The narrow bore of the capillary (less than 300 µm, advantageously 250 µm) of the test section and its length of less than 200 mm, (advantageously 152 mm) ensures that at suitable fuel flow rates of e.g. 35 ml/min that the fuel is in the turbulent flow regime. The thermal degradation of test fuel within the test section is advantageously monitored in two ways: (1) A computer controlled scanning I.R. thermometer 4 measures outer wall temperatures along the test section 2 and subsequent increases in these temperature, promoted by the insulation effect of fuel degradation products; (2) Fuel degradation products are mostly carbon by mass, thus a carbon analyser (not shown) can assess the weight of carbon deposited.

A fuel of low stability will produce a high weight of carbon deposition (e.g. 200 µg) and a high rise in outer wall temperature (e.g. 300 °C rise 10 mm from fuel outlet). A fuel of good stability will produce a much lower weight of carbon deposition (e.g. 10-20 µg) and a lower rise in outer wall temperature (e.g. 20 °C rise 10 mm from fuel outlet).

The ability to monitor accurately the amount of deposit within an extremely narrow bore has been achieved by scanning I.R. thermometry of the outer wall surface temperature. As fuel lacquer is deposited it insulates the hot metal from the colder fuel, thus the outer wall temperature increases during the test.

Contact systems of temperature measurement on the test section (e.g. thermocouples) are not viable, either because of the large heat drain caused to the test section or alternatively damage to the small diameter-test section by attaching thermocouples.

The use of an I.R. thermometer 4 enables the surface temperature of the test section 2 to be measured without contact. The test section 2 is e.g painted matt black to ensure a constant and high emissivity (close to 0.9). As already indicated in the foregoing, the fuel enters the test section 2 at a constant fuel inlet temperature and the heat applied to the test section 2 is controlled to a constant fuel outlet temperature via fuel temperature control 3 and any suitable temperature control program via connection line 5. As fuel deposits on the inside of the capillary it insulates the hotter metal from the colder fuel, i.e. it increases the thermal resistance at that point. The change in surface temperature from the start of test (ΔT) is a measure of the thermal resistance, which in turn is a measure of the fuel lacquer thickness.

Thus, to determine the lacquer thickness within the capillary test section along its length, ΔT measurements are determined at several positions along the capillary test section. This is achieved by a computer (6) controlled system which scans by means of any suitable scanner control program via connection line 7 the I.R. thermometer 4 along the capillary 2 throughout the test, the temperature data so derived is logged on the same computer 6 throughout the test. The arrow C represents the scanner movement. Data D can be captured from the computer 6 and can be processed further in any way suitable for the purpose.

E.g in this manner a "map" of the lacquer pattern along the length can be obtained, and this is a reliable way of distinguishing fuels of different stability. Such a "map" provides a three dimensional temperature profile of the capillary, resolved in position and time. Reference is made to fig. 2. In fig. 2 the vertical axis (Z) represents ΔT (in deg. C), and the horizontal axes (X and Y) represent distance from the fuel outlet (in mm) and the time (in min.) respectively. The parts of different shading represent differences in profile temperatures. Each block of a particular shade represents the delta T (temperature) profile along the capillary for a given time, e.g. the first block at the front represents the delta T profile at zero time, the next shaded block immediately behind it represents the profile 10 minutes into the test. And so on, until the shaded block at the very back of the picture is the profile at the end of test (120 minutes in this example). The method of the invention will now be illustrated by reference to the following example:

The system uses advantageously a 254 micron (1/100 inch) diameter capillary with a pump delivering fuel at a rate of advantageously 35 ml/min. In practice, a 4.2 litre fuel sample is required in a 2 hour test.

Along the capillary test section (advantageously 6 inches, 152 mm length) there is a marked fuel temperature gradient, fuel enters advantageously at ambient temperature (15-25 °C, in particular at 20 °C) and exits advantageously at 280-300 °C, in particular at 290 °C. There is thus a Reynold's number gradient along the test section due to the change in kinematic viscosity. At the fuel inlet the Reynold's number is circa 2500 and at the exit circa 14000, the average down the tube would be a Reynold's number of 8500 corresponding to a 170 °C fuel temperature. All monitoring of fuel deposition is advantageously within 21 mm of the fuel outlet, so only fuel deposition in the turbulent flow regime is followed.

There is little scope for changing the capillary dimensions and yet at the same time maintain the use of a small sample and have measurable changes in heat transfer/thermal resistance:-
- For a given Reynold's number, halving the bore would increase the pressure drop across the test section from 350 PSI (about 25 bar) to 2800 PSI (about 196 bar), it would be difficult to get a pumping system for these pressures.
- For a given Reynold's number, double the bore diameter would double the fuel sample to 8.4 litres, this is too large if the apparatus is to look at small samples from the field.
- The larger diameter would also mean that changes in heat transfer to measure deposit levels would be harder to discern. (Indeed they may not even be possible in the current system).

The scanning system software allows changes in the scanning routine but most of the fuel deposition is in the region 1-21 mm from the fuel outlet, so the scan advantageously reads a determined number of positions (e.g. nine) between 1 and 21 mm from the fuel outlet. Each scan of a single position includes a certain time wait (e.g. 10 seconds) (for stabilisation of the I.R. thermometer) then the computer averages a determined number of readings e.g ten over a determined number of seconds (e.g. ten) from this one position, before moving onto the next position. In this case all nine positions form the scan sequence which take circa 2.5 minutes to perform. A sequence of scans starts e.g. every five minutes, so in the 120 minute test there are 24 scan sequences.

## Claims

1. A method for testing the thermal stability of a fuel comprising the steps of
a) supplying under pressure a fuel sample to be tested to a small-bore capillary tube of a test section and allowing the fuel in the test section to be heat stressed;
b) ensuring that the fuel sample in the test section is under turbulent flow regime;
c) monitoring the thermal degradation of the test fuel within the section by measuring by means of contactless thermometry the outer wall temperatures along the test section and subsequent increases in these temperatures, promoted by the insulation effect of fuel degradation products; and
d) deriving from the temperature data, thus obtained, information on the thermal stability of the fuel.

2. The method as claimed in claim 1, wherein the contactless thermometry is controlled scanning I.R. (infra-red) thermometry.

3. The method as claimed in claims 1 or 2, wherein fuel is deposited on the inside of the capillary tube at a determined distance from the fuel outlet, advantageously at 1-21 mm.

4. The method as claimed in claims 2 or 3, wherein the I.R. thermometry scan reads a determined number of positions, advantageously 9, along the capillary test section.

5. The method as claimed in claim 4, wherein each I.R. thermometry scan of a position includes a determined time wait, advantageously 10 sec.

6. The method as claimed in claim 5, wherein a determined number of readings, advantageously 10, are carried out over a determined number of seconds, advantageously 10, from one position before moving to the next position.

7. The method as claimed in any one of claims 2-6, wherein a sequence of scans is carried out.

8. The method as claimed in any one of claims 1-7, wherein the fuel sample consumption is less than 5 litre per test.

9. The method as claimed in any one of claims 1-8, comprising the step of assessing the weight of carbon deposit.

10. The method as claimed in any one of claims 1-9, wherein the fuel enters at 15-25 °C, in particular 20 °C and exits at 280-300 °C, in particular 290 °C.

11. An apparatus for testing the thermal stability of a fuel comprising means for supplying under pressure a fuel sample to be tested to a small-bore capillary tube of a test section and means for allowing the fuel in the test section to be heat stressed; means for ensuring that the fuel sample in the test section is under turbulent flow regime; means for measuring by means of contactless thermometry the outer wall temperatures along the test section and subsequent increases in these temperatures, promoted by the insulation effect of fuel degradation products; and means for deriving from the temperature data, thus obtained, information on the thermal stability of the fuel.

12. The apparatus as claimed in claim 11, wherein means for controlled scanning I.R. (infra-red) thermometry are present.

13. The apparatus as claimed in claim 11 or 12, wherein the capillary tube has a diameter of less than 300 µm.

14. The apparatus as claimed in any one of claims 11-13, wherein the capillary test section has a length of less than 200 mm.

15. The apparatus as claimed in any one of claims 11-14, wherein a carbon analyzer is present to assess the weight of carbon deposited.

## Patentansprüche

1. Verfahren zum Prüfen der thermischen Stabilität eines Kraftstoffes, mit den Schritten
a) Zuführen einer zu testenden Kraftstoffprobe unter Druck zu einem Kapillarrohr kleiner Bohrung eines Testabschnittes, und Zulassen, daß der Kraftstoff in dem Testabschnitt wärmebeansprucht wird;
b) Gewährleisten, daß sich die Kraftstoffprobe im Testabschnitt unter Turbulenzströmungsbedingungen befindet;
c) Überwachen des thermischen Abbaus des Testkraftstoffes in dem Abschnitt durch Messen der Außenwandtemperaturen entlang des Testabschnittes und darauffolgender Anstiege dieser Temperaturen, die durch den Isolierungseffekt der Kraftstoffabbauprodukte hervorgerufen werden, mittels berührungsloser Temperaturmessung; und
d) Ableiten von Information über die thermische Stabilität des Kraftstoffes aus den so erhaltenen Temperaturdaten .

2. Verfahren nach Anspruch 1, bei welchem die berührungslose Temperaturmessung eine gesteuert abtastende Infrarottemperaturmessung ist.

3. Verfahren nach Anspruch 1 oder 2, bei welchem sich Kraftstoff an der Innenseite des Kapillarrohres in einem vorgegebenen Abstand vom Kraftstoffauslaß ablagert, bevorzugt bei 1-21 mm.

4. Verfahren nach Anspruch 2 oder 3, bei welchem die Infrarottemperaturabtastung eine vorgegebene Anzahl von Positionen, bevorzugt 9, entlang des kapillaren Testabschnittes mißt.

5. Verfahren nach Anspruch 4, bei welchem jede Infrarottemperaturabtastung einer Position eine vorgegebene Wartezeit inkludiert, bevorzugt 10 s.

6. Verfahren nach Anspruch 5, bei welchem eine vorgegebene Anzahl von Messungen, bevorzugt 10, über eine vorgegebene Anzahl von Sekunden, bevorzugt 10, von einer Position durchgeführt wird, bevor zur nächsten Position übergegangen wird.

7. Verfahren nach einem der Ansprüche 2-6, bei welchem eine Folge von Abtastungen ausgeführt wird.

8. Verfahren nach einem der Ansprüche 1-7, bei welchem der Kraftstoffprobenverbrauch weniger als 5 Liter pro Test ist.

9. Verfahren nach einem der Ansprüche 1-8, mit dem Schritt des Bestimmens des Gewichtes der Kohlenstoffablagerung.

10. Verfahren nach einem der Ansprüche 1-9, bei welchem Kraftstoff mit 15-25°C eintritt, insbesondere 20°C, und mit 280-300°C, insbesondere 290°C, austritt.

11. Vorrichtung zum Prüfen der thermischen Stabilität eines Kraftstoffes, mit Mitteln zum Zuführen einer zu testenden Kraftstoffprobe unter Druck zu einem Kapillarrohr kleiner Bohrung eines Testabschnittes, und Mitteln zum Zulassen einer Wärmebeanspruchung des Kraftstoffes im Testabschnitt; Mitteln zum Gewährleisten, daß die Kraftstoffprobe im Testabschnitt unter Turbulenzströmungsbedingungen steht; Mitteln zum Messen der Auβenwandtemperaturen entlang des Testabschnittes und darauffolgender Anstiege dieser Temperaturen, welche durch den Isolationseffekt der Kraftstoffabbauprodukte hervorgerufen werden, mittels berührungsloser Temperaturmessung; und Mitteln zum Ableiten von Information über die thermische Stabilität des Kraftstoffes aus den so erhaltenen Temperaturdaten.

12. Vorrichtung nach Anspruch 11, bei welcher Mittel zur gesteuert abtastenden Infrarotthemperaturmessung vorhanden sind.

13. Vorrichtung nach Anspruch 11 oder 12, bei welcher das Kapillarrohr einen Durchmesser von weniger als 300 µm hat.

14. Vorrichtung nach einem der Ansprüche 11-13, bei welcher der kapillare Testabschnitt eine Länge von weniger als 200 mm hat.

15. Vorrichtung nach einem der Ansprüche 11-14, bei welcher ein Kohlenstoffanalysator vorhanden ist, um das Gewicht des abgelagerten Kohlenstoffes zu bestimmen.

## Revendications

1. Procédé pour tester la stabilité thermique d'un carburant comprenant les étapes consistant :
a) à amener sous pression un échantillon de carburant à tester dans un tube capillaire de petit calibre d'une section d'essai et à soumettre le carburant dans la section d'essai à une contrainte thermique;
b) à s'assurer que l'échantillon de carburant dans la section d'essai soit sous un régime d'écoulement turbulent;
c) à contrôler la dégradation thermique du carburant d'essai dans la section en mesurant au moyen d'une thermométrie sans contact les températures de paroi extérieure le long de la section d'essai et les élévations ultérieures de ces températures, favorisées par l'effet d'isolement des produits de dégradation du carburant; et
d) à déduire des données de température, ainsi obtenues, une information sur la stabilité thermique du carburant.

2. Procédé suivant la revendication 1, dans lequel la thermométrie sans contact est une thermométrie I.R. (infrarouge) à balayage commandée.

3. Procédé suivant l'une ou l'autre des revendications 1 et 2, dans lequel le carburant est déposé à l'intérieur du tube capillaire à une distance déterminée de la sortie de carburant, avantageusement à 1-21 mm.

4. Procédé suivant l'une ou l'autre des revendications 2 et 3, dans lequel le balayage de la thermométrie I.R. lit un nombre déterminé de positions, avantageusement 9, le long de la section d'essai du tube capillaire.

5. Procédé suivant la revendication 4, dans lequel chaque balayage de la thermométrie I.R. d'une position inclut un temps d'attente déterminé, avantageusement 10 secondes.

6. Procédé suivant la revendication 5, dans lequel un nombre déterminé de lectures, avantageusement dix, sont réalisées sur un nombre déterminé de secondes, avantageusement dix, d'une position avant de passer à la position suivante.

7. Procédé suivant l'une quelconque des revendications 2 à 6, dans lequel une séquence de balayages est réalisée.

8. Procédé suivant l'une quelconque des revendications 1 à 7, dans lequel la consommation d'échantillon de carburant est inférieure à 5 litres par essai.

9. Procédé suivant l'une quelconque des revendications 1 à 8, comprenant l'étape de détermination du poids de dépôt de carbone.

10. Procédé suivant l'une quelconque des revendications 1 à 9, dans lequel le carburant entre à 15-25°C, en particulier 20°C et sort à 280-300°C, en particulier 290°C.

11. Appareil pour tester la stabilité thermique d'un carburant, comprenant un moyen pour amener sous pression un échantillon de carburant à tester dans un tube capillaire de petit calibre d'une section d'essai et un moyen pour soumettre le carburant dans la section d'essai à une contrainte thermique, un moyen pour s'assurer que l'échantillon de carburant dans la section d'essai soit sous un régime d'écoulement turbulent, un moyen pour mesurer au moyen d'une thermométrie sans contact les températures de paroi extérieure le long de la section d'essai et les élévations ultérieures de ces températures, favorisées par l'effet d'isolement des produits de dégradation du carburant et un moyen pour déduire des données de température, ainsi obtenues, une information sur la stabilité thermique du carburant.

12. Appareil suivant la revendication 11, dans lequel un moyen pour une thermométrie I.R. (infrarouge) à balayage commandée est présent.

13. Appareil suivant l'une ou l'autre des revendications 11 et 12, dans lequel le tube capillaire a un diamètre inférieur à 300 µm.

14. Appareil suivant l'une quelconque des revendications 11 à 13, dans lequel la section d'essai du tube capillaire a une longueur inférieure à 200 mm.

15. Appareil suivant l'une quelconque des revendications 11 à 14, dans lequel un analyseur de carbone est présent pour déterminer le poids de carbone déposé.
